(19) Europäisches Patentamt
European Patent Office
Office européen des brevets

(11) Publication number: **0 375 082 B1**

(12) # EUROPEAN PATENT SPECIFICATION

(45) Date of publication of patent specification: **08.03.95**

(51) Int. Cl.[6]: **A61K 7/06**, A61K 7/48

(21) Application number: **89203315.0**

(22) Date of filing: **22.12.89**

(54) **Cosmetic composition.**

(30) Priority: **22.12.88 JP 321940/88**
**26.09.89 JP 249983/89**

(43) Date of publication of application:
**27.06.90 Bulletin 90/26**

(45) Publication of the grant of the patent:
**08.03.95 Bulletin 95/10**

(84) Designated Contracting States:
**AT CH DE ES FR GB IT LI**

(56) References cited:
**EP-A- 0 245 871**
**EP-A- 0 255 937**
**EP-A- 0 283 713**
**DE-C- 725 292**
**FR-A- 2 466 273**
**GB-A- 505 983**

**PATENT ABSTRACTS OF JAPAN, vol. 10, no. 383 (C-393)[2440], 23rd December 1986;& JP-A-61 176 511 (KANEBO) 08-08-1986**

**Römmps Chemie Lexikon (1983), p. 2338-2339**

(73) Proprietor: **KANEBO LTD.**
**No. 17-4 Sumida 5-chome**
**Sumida-ku**
**Tokyo (JP)**

(72) Inventor: **Togiya, Satoshi**
**879-9, Kuno**
**Odawara City**
**Kanagawa Prefecture (JP)**
Inventor: **Yamada, Satomi**
**3-19-28-505, Jukkenzaka**
**Chigasaki City**
**Kanagawa Prefecture (JP)**
Inventor: **Kondo, Mitsuo**
**188-6, Nakazato**
**Odawara City**
**Kanagawa Prefecture (JP)**

(74) Representative: **Smulders, Theodorus A.H.J., Ir. et al**
**Vereenigde Octrooibureaux**
**Nieuwe Parklaan 97**
**NL-2587 BN 's-Gravenhage (NL)**

EP 0 375 082 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

### Field of the Invention

This invention relates to a cosmetic composition, particularly those containing at least one saponin in combination with monoacyl phosphatide.

### Description of the Prior Art

There has recently been much research on solubilization. As a result, improvement is seen in solubilizers as well as technology of solubilization. Now, stable solubilized materials are being used in many fields.

Most of those solubilizers are nonionic surface active agents having polyoxyethylene chains, anionic surface active agents such as fatty acid soaps, cationic surface active agents and amphoteric surface active agents. However, solubilized clear cosmetics in which those synthetic surface active agents are used generally have the drawback that they often stimulate the skin and hair.

Under the above circumstances, phosphatide, particularly monoacyl phosphatide, has drawn attention. Japanese Patent Application Laid-Open 112007/86 discloses clear cosmetic compositions in which monoacyl phosphatide is used as a solubilizer. Japanese Patent Application Laid-Open 171407/86 discloses emulsion cosmetic compositions in which monoacyl phosphatide is used as an emulsifier.

However, the previous clear cosmetic compositions in which monoacyl phosphatide is used have a problem with storage stability, particularly in low and high temperature conditions. In addition, further improvement is needed in organoleptic properties, particularly continued smoothness, and appearance such as texture and clearness.

That is, when clear cosmetic compositions are stored at a low temperature (below 5 °C) or high temperature (above 40 °C), monoacyl phosphatide tends to precipitate or cause turbidity.

Meanwhile, saponin is a compound in which a nonpolar group (saponigen) such as sterol or triterpene is bound to saccharide such as pentose, hexose or uronic acid. They are found broadly in various plants besides certain types of asteroids. More than one type of saponin often exists in a single plant. Saponin has been studied as a medicamental component in recent years, but only a few cosmetic compositions are known in which saponin is used. It is suggested to use a saponin component of licorice, i.e., glycyrrhizin, in a cosmetic composition from a viewpoint of its anti-inflammatory effect, anti-allergic effect, antigen antibody suppression effect and steroid hormonic effect (Fragrance Journal, Vol. 3, No. 4, 39 - 41, 1975, Japan). It is known that in a skin cosmetic containing an oil obtained from seed of Oenothera tetraptera Cav., an extract from seed of Helianthus annuus L., root of Bupleurum falcatum L. or rhizome of Sanguisorba officinalis L. is blended as a stabilizer to prevent acidification or oxidation of the oil, and the extract contains saponin (Japanese Patent Application Laid-Open 178908/86). A shampoo is known in which a main component is an aqueous extract from pericarp of Sapindus mukurosii. This is said to have practically sufficient foaming ability and detergency (Japanese Patent Application Laid-Open 125510/77). A hair tonic containing an extract from crude drugs such as Chinese matrimony vine is known, in which polyoxyethylene-hardened castor oil, polyoxyethylene sorbitan sesquioleate or polyoxyethylene sorbitan monostearate is used as an emulsifier or solubilizer (Japanese Patent Publication 40923/83).

U.S. Patent 3,652,397 discloses a process for the preparation of a lisophosphatide emulsifying agent by partially hydrolysing phosphatide obtained from soyabean, etc. This emulsifying agent contains unreacted phosphatide, i.e. diacyl phosphatide, besides the reaction product lysophosphatide, i.e. monoacyl phosphatide. The above U.S. Patent 3,652,397 discloses the application of the lysophosphatide emulsifying agent mostly in foodstuffs such as margarine and mayonaise. Except for just one line, "cosmetic preparations, for example lotions and salvers", there is no concrete disclosure on cosmetic compositions. In reality, clear lotions cannot be obtained by the use of diacyl phosphatide. The U.S. Patent does not suggest that emulsion cosmetic compositions in which a combination of monoacyl phosphatide and diacyl phosphatide is used, show unexpectedly high stability during storage, improved organoleptic properties and appearance.

### Summary of the Invention

The present inventors have conducted keen research to solve the aforesaid drawback of cosmetic compositions in which monoacyl type phosphatide is used and have now found that when monoacyl type phosphatide is used in combination with saponin, precipitation or turbidity does not occur during storage,

and organoleptic properties and appearance are improved.

The effects attained by the above combination could not be expected.

Thus, the present invention provides a cosmetic composition comprising an oily substance, water, a phosphatide and saponin, characterized in that the composition is a clear composition wherein the phosphatide is at least one monoacyl type phosphatide represented by the following general formula (1) or (2):

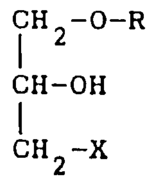

(1)

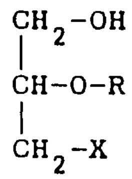

(2)

wherein R represents

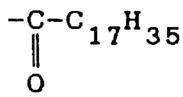 or 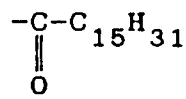,

and X represents

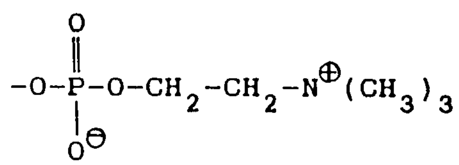,

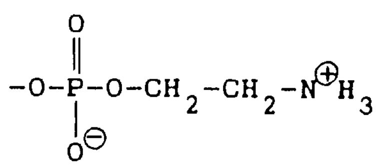 or

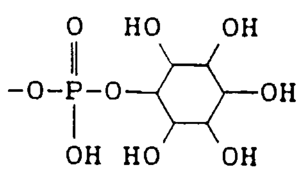.

Detailed Description of the Invention

The compounds represented by the aforesaid general formula (1) or (2) in the invention, i.e. mono-O-acyl-3-glyceryl phosphoryl choline, mono-O-acyl-3-glyceryl phosphoryl ethanolamine and mono-O-acyl-3-glyceryl phosphoryl inositol, are publicly known per se. The compounds represented by the general formula (1) include 1-palmitoyl-3-glycerylphosphoryl choline, 1-stearoyl-3-glyceryl phosphoryl choline, 1-palmitoyl-3-glyceryl phosphoryl ethanolamine, 1-stearoyl-3-glyceryl phosphoryl ethanolamine, 1-palmitoyl-3-glyceryl phosphoryl inositol, and 1-stearoyl-3-glyceryl phosphoryl inositol. The compounds represented by the general formula (2) include 2-palmitoyl-3-glycerylphosphoryl choline, 2-stearoyl-3-glyceryl posphoryl choline, 2-palmitoyl-3-glyceryl phosphoryl ethanolamine, 2-stearoyl-3-glyceryl phosphoryl ethanolamine, 2-palmitoyl-3-glyceryl phosphoryl inositol, and 2-stearoyl-3-glyceryl phosphoryl inositol.

The compounds represented by the general formula (1) or (2) may be prepared by treating a starting material phosphatidyl choline, phosphatidyl ethanolamine or phosphatidyl inositol obtained from, for instance, yolk with snake venom phospholipase or enzyme extracted from swine pancreas, i.e. pancreatin, and then fractioning it by high performance liquid chromatography. Alternatively, the compounds may be chemically synthesized.

The compounds represented by the general formula (1) or (2) are safe to a human body. When their stimulus to skin was tested in accordance with Draize's method, the score of animal skin stimulus and the score of human skin stimulus were both zero, which means that they are non-stimulative, [Draize, J. H., Association of Food and Drug officials of the United States. "Appraisal of the Safety of Chemicals in Foods, Drugs and Cosmetics", 46(1959), Texas, State Department of Health, Austin.]

One or more of the compounds represented by the above general formula (1) or (2) according to the invention may be used alone or in combination as a solubilizer.

Saponin or saponin-containing plant extracts used in the invention are publicly known.

As stated above, saponin used in the invention is defined as a compound in which a nonpolar group (i.e. saponigen) such as sterol or triterpene is bound to saccharide such as pentose, hexose or uronic acid. They are found broadly in various plants besides certain types of asteroids. More than one type of saponin exists in a single plant. Although the present invention should not be particularly limited by their sources or acquisition methods, saponin-containing sources include Ophiopogonis tuber, Asparagi radix, Phytolaccae radix, Senegae radix, Polygalae radix, Ginseng radix (Pharmacia, Vol. 15, No. 10, 897 - 899, 1979, Japan), Glycyne max Merrill (Japanese Patent Application Laid-Open 160981/81), Gynostenma pentaphyllum MAKINO (Japanese Patent Application Laid-Open 127316/81), licorice (Fragrance Journal, Vol. 3, No. 4, 39 - 41, 1975, Japan), seed of Hetianthus annuus L., root of Bupleurum falcatum L., rhizome of Sanguisorba officinalis L. (Japanese Patent Application Laid-Open 178908/89), pericarp of Sapindus mukurosii (Japanese Patent Application Laid-Open 125510/77), roots of Bupleurum falcatum L., etc. (Japanese Patent Publication 10923/67), Cirsium mill, Cinese matrimony vine, Bupleurum falcatum L. and Cnidium officinale Makino (Japanese Patent Publication 40923/83), senega, Hamamelis virginiana, Potentilla wallichiana Del. and Althaea officinalis (Japanese Patent Application Laid-Open 106607/82). In the present invention, saponin-containing plant extracts obtained by extracting the aforesaid plants in any known method, or purified saponin therefrom may be used.

Particularly preferred examples include saponin obtained from licorice ($\alpha$- or $\beta$-glycyrrhizin or derivatives thereof), an extract from pericarp of Sapindus mukurosii, soyabean saponin, extract from rhizome of Sanguisorba officinalis L., extract from seed of tea trees, and extract from seed of camellia.

Clear cosmetic compositions may be prepared using a combination of the compound represented by the above formula (1) or (2) with saponin.

The amount of the compound represented by the above formula (1) or (2) used and the amount of the above saponin or saponin-containing extract (as a saponin content) used are both about 0.01 to about 10 % by weight, preferably 0.1 to 5 % by weight, based on the total weight of a cosmetic composition.

If even either of the compound of formula (1) or (2) and saponin is used in an amount less than the aforesaid amount, storage stability of a cosmetic composition is poor. On the other hand, if they are used in an amount larger than the above amount, they do not completely dissolve in the system and tend to separate, and further the feel of the use of a cosmetic composition tends to worsen.

The terms "oily substance" as used herein mean, generally, oily substance, such as oil-soluble perfumes, oil-soluble vitamins, oil-soluble hormones, oil-soluble colors, oil-soluble UV absorbers, higher aliphatic hydrocarbons, vegetable oils, animal oils, waxes, higher fatty acids, higher alcohols and synthetic ester oils.

More specifically, the oil-soluble perfumes include those extracted from natural animals or plants and synthetic ones. The oil-soluble vitamins include vitamins A, D, E, F and K, vitamin derivatives such as

pyridoxine dicaprylate, pyridoxine dipalmitate, dl-α-tocopherol acetate, dl-α-tocopherol nicotinate, ascorbyl dipalmitate, ascorbyl monopalmitate and ascorbyl monostearate. The oil-soluble hormones include estradiol, ethinyl estradiol, estrone and diethylstilbestrol. The oil-soluble colors inculde Sudan III, tetrabromofluoresceine, dibromofluoresceine, fluoresceine and quinizarin green SS. The oil-soluble UV absorbers include oxybenzone and methyl 2,5-diisopropyl cinnamate.

The higher aliphatic hydrocarbons include liquid paraffin, squalane, microcrystalline wax, vaseline and ceresin. The vegetable oils include olive oil, castor oil, cacao oil and palm oil. The animal oils include cod liver oil, beef tallow and butter oil. The waxes include bees wax and carnauba wax. The higher fatty acids include lauric acid, myristic acid, palmitic acid, stearic acid, oleic acid, behenic acid and lanolin fatty acid. The higher alcohols include lauryl alcohol, stearyl alcohol, cetyl alcohol and oleyl alcohol. The synthetic ester oils include linear or branched esters such as butyl stearate, hexyl laurate, octyl dodecyl myristate, diisopropyl adipate and diisopropyl sebacate.

One or more of these oily substances may be used alone or in combination.

In the clear cosmetic compositions such as lotions and hair tonics, the used amount of the oily substance ranges from about 0.0001 to about 1 % by weight, preferably from about 0.001 to about 0.3 % by weight, based on the total weight of whole formulation. If the amount is less than about 0.0001 % by weight, intrinsic effects of the oily substance in question are insufficiently exhibited. If it exceeds about 1 % by weight, the substance tends to be difficultly solubilized.

To the cosmetic composition according to the invention may be added humectants, medicinal components for beauty, perfumes, antiseptics, colorants, UV absorbers, astringents, synthetic surface active agents, pigments such as kaolin, mica, sericite, talc, yellow iron oxide and red iron oxide, water-soluble natural high molecular weight substances such as casein soda, pectine, xanthane gum, karaya gum, locust bean gum and carrageenan, if needed.

Examples of the cosmetic composition according to the invention include skin treatment lotions, facial fresheners, softening lotions, acne treatment lotions, after-shave lotions, conditioning lotions containing pigments or powder, cleansing lotions, hair tonics, but the invention is not limited to these.

## Examples

Preferred embodiments of the invention will be explained below.

"Part" hereinafter means part by weight.

The properties of cosmetic compositions examined in the Examples were storage stability, organoleptic properties, pH, clearness and appearance. The testing methods are as follows.

### (1) Storage stability

Sample compositions are allowed to stand in a thermostatic chamber at a temperature of 45 °C, or 0 °C for 3 months, and the presence of precipitates is examined.

### (2) Organoleptic properties

Feels of the application such as refreshing feel, moistening feel, greasy feel and smoothness, and finishing after the application are examined by three specialists and overall evaluation is made.

### (3) Determination of pH

A pH value is determined immediately after the preparation of a cosmetic composition. A pH-measuring unit with a glass electrode is well adjusted using a standard pH liquid and then used.

### (4) Clearness

Clearness is expressed by transmittance of light of 450 nm wave length. When the value is 80 % or higher, a substance is visually transparent.

### (5) Appearance

Color or texture is visually evaluated.

Examples 1 to 5 and Comparison Examples 1 and 2

Cosmetic lotions were prepared with the formulations shown in Table 1 (part by weight). 1-Palmitoyl-3-glyceryl phosphoryl choline was used as phosphatide. The used licorice saponin was commercially bought and contained α-glycyrrhizin mainly.

Ingredients 1 to 3 shown in Table 1 were mixed to dissolve, which was called solution 1. Separately, ingredients 4 to 7 shown in Table 1 were mixed to dissolve, which was called solution 2. Solutions 2 was admixed to solution 1 to prepare a cosmetic lotion. The properties of the resulting cosmetic lotion are as shown in Table 1.

As seen from the Table, the cosmetic lotions which contained both phosphatide and saponin as the Examples of the invention showed excellent storage stability. In contrast, the cosmetic lotions of Comparison Examples 1 and 2 which lacked either one of phosphatide or saponin showed poor storage stability to cause precipitation, turbidity and floating oil. The cosmetic lotions of the invention were superior to those of the Comparison Examples also in the organoleptic properties and clearness.

Table 1

| Ingredient | | Ex.1 | Ex.2 | Ex.3 | Ex.4 | Ex.5 | Comp.1 | Comp.2 |
|---|---|---|---|---|---|---|---|---|
| 1. 1-Palmitoyl-3-glyceryl phosphoryl choline | | 0.01 | 0.1 | 1.0 | 5.0 | 10.0 | - | 0.1 |
| 2. Ethanol | | 10.0 | 10.0 | 10.0 | 10.0 | 10.0 | 10.0 | 10.0 |
| 3. Bergamot oil | | 0.04 | 0.04 | 0.04 | 0.04 | 0.04 | 0.04 | 0.04 |
| 4. Dipropylene glycol | | 5.0 | 5.0 | 5.0 | 5.0 | 5.0 | 5.0 | 5.0 |
| 5. Licorice saponine (α-glycyrrhizin) | | 0.1 | 0.1 | 0.1 | 0.1 | 0.1 | 0.1 | - |
| 6. Disodium hydrogen-phosphate | | 0.012 | 0.008 | 0.003 | 0.022 | 0.067 | 0.015 | 0.002 |
| 7. Purified water | | 84.838 | 84.752 | 83.857 | 79.838 | 74.793 | 84.845 | 84.858 |
| Storage stability (precipitation) | 0℃, 3 months | G | G | G | G | G | T | T |
| | 45℃, 3 months | G | G | G | G | G | O | P |
| Organoleptic properties | | G | G | G | G | Slightly G | Slightly G | Greasy |
| Clearness | immediately After preparation | 82 | 87 | 92 | 91 | 86 | 71 | 78 |
| | 6 months after, 30℃ | 80 | 86 | 93 | 90 | 82 | 60 | 63 |
| pH | | 5.5 | 5.5 | 5.5 | 5.5 | 5.5 | 5.5 | 5.5 |

G: Good, T: Turbid, O: Floating Oil, P: Precipitation

## Claims

**1.** A cosmetic composition comprising an oily substance, water, a phosphatide and saponin, characterized in that the composition is a clear composition wherein the phosphatide is at least one monoacyl type

phosphatide represented by the following general formula (1) or (2):

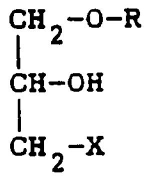

(1)

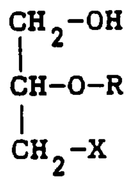

(2)

wherein R represents

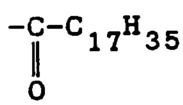 or 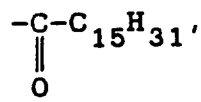

and X represents

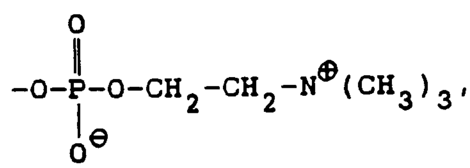

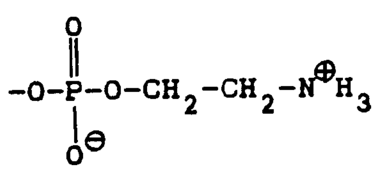 or

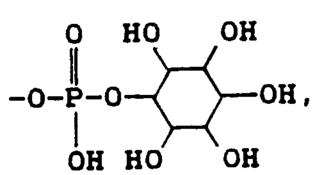

**2.** The cosmetic composition according to claim 1, wherein the saponin is one obtained from licorice, pericarp of Sapindus mukurosii, soyabean, rhizome of Sanguisorba officinalis L., seed of tea trees or seed of camellia, or derivatives thereof.

**3.** The cosmetic composition according to claim 1, wherein the saponin is added in the form of a saponin-containing plant extract.

**4.** The cosmetic composition according to any of claims 1-3, wherein the saponin and the monoacyl type phosphatide are each contained in an amount of 0.01 to 10% by weight, based on the total weight of the cosmetic composition.

**5.** The cosmetic composition according to claim 4, wherein the saponin and the monoacyl type phosphatide are each contained in an amount of 0.1 to 5 % by weight, based on the total weight of the cosmetic composition.

**6.** The cosmetic composition according to any of claims 1-5, wherein the clear cosmetic composition is selected from skin treatment lotions, facial fresheners, softening lotions, acne treatment lotions, after-shave lotions, conditioning lotions containing pigments or powder, cleansing lotions and hair tonics.

**Patentansprüche**

**1.** Eine kosmetische Zusammensetzung, die eine ölige Substanz, Wasser, ein Phosphatid und Saponin umfaßt, dadurch gekennzeichnet, daß die Zusammensetzung eine durchsichtige Zusammensetzung ist, in der das Phosphatid zumindest ein Phosphatid vom Monoacyl-Typ ist, welches durch die folgenden allgemeinen Formeln (1) und (2) dargestellt wird,

$$\begin{array}{l} CH_2-O-R \\ | \\ CH-OH \\ | \\ CH_2X \end{array} \qquad (1)$$

$$\begin{array}{l} CH_2-OH \\ | \\ CH-O-R \\ | \\ CH_2-X \end{array} \qquad (2)$$

worin R

$$\begin{array}{l} -\underset{\underset{O}{\|}}{C}-C_{17}H_{35} \end{array} \quad \text{oder} \quad \begin{array}{l} -\underset{\underset{O}{\|}}{C}-C_{15}H_{31} \end{array}$$

und und X

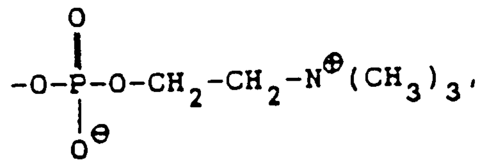

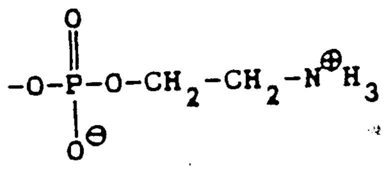

oder

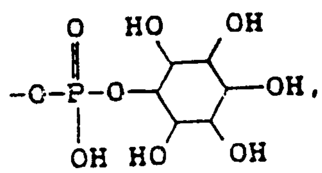

bedeuten.

2. Die kosmetische Zusammensetzung gemäß Anspruch 1, worin das Saponin ein solches ist, das aus Lakritze, Fruchthüllen von Sapindus mukurosii, Sojabohnen, Rhizomen von Sanguisorba officinalis L., Samen vom Teestrauch oder Samen der Kamelie erhalten wird, oder Derivate hiervon.

3. Die kosmetische Zusammensetzung gemäß Anspruch 1, worin das Saponin in Form eines saponinhaltigen Pflanzenextrakts zugegeben wird.

4. Die kosmetische Zusammensetzung gemäß jedem der Ansprüche 1-3, worin das Saponin und das Phosphatid vom Monoacyl-Typ beide in einer Menge von 0,01 bis 10 Gew.%, bezogen auf das Gesamtgewicht der kosmetischen Zusammensetzung, enthalten sind.

5. Die kosmetische Zusammensetzung gemäß dem Anspruch 4, worin das Saponin und das Phosphatid vom Monoacyl-Typ beide in einer Menge von 0,1 bis 5 Gew.%, bezogen auf das Gesamtgewicht der kosmetischen Zusammensetzung, enthalten sind.

6. Die kosmetische Zusammensetzung gemäß jedem der Ansprüche 1-5, worin die durchsichtige kosmetische Zusammensetzung aus Hautbehandlungslotionen, Gesichtswässern, Weichhaltungslotionen, Lotionen zur Aknebehandlung, Aftershave-Lotionen, Konditionierungslotionen, die Pigmente oder Puder enthalten, Reinigungslotionen und Haartonika ausgewählt ist.

**Revendications**

1. Composition cosmétique comprenant une substance huileuse, de l'eau, un phosphatide et de la saponine, cette composition étant caractérisée en ce qu'elle est une composition limpide dans laquelle le phosphatide est au moins un phosphatide de type monoacyle représenté par la formule générale (1) ou (2) suivante :

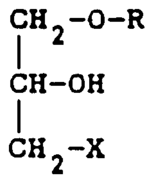

(1)

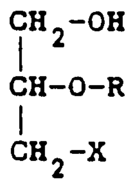

(2)

où R représente

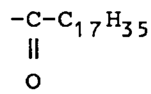 ou 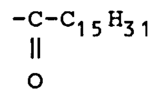

et X représente

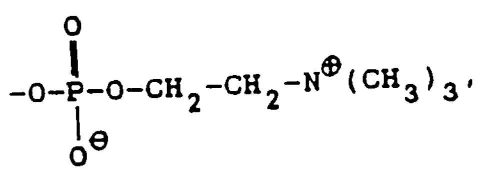

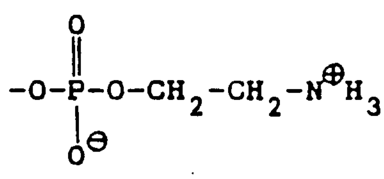 ou 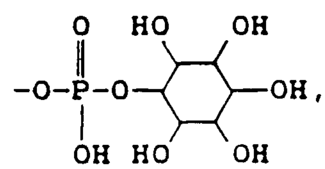

2. Composition cosmétique selon la revendication 1, dans laquelle la saponine est obtenue à partir de réglisse, de péricarpe de Sapindus mukurosii, de soja, de rhizome de Sanguisorba officinalis L., de graines de théier ou de graines de camélia ou de leur dérivés.

3. Composition cosmétique selon la revendication 1, dans laquelle la saponine est ajoutée sous forme d'un extrait végétal contenant de la saponine.

4. Composition cosmétique selon l'une quelconque des revendications 1 à 3, dans laquelle la saponine et le phosphatide de type monoacyle sont contenus chacun en une proportion de 0,01 à 10 % en poids, relativement au poids total de la composition cosmétique.

5. Composition cosmétique selon la revendication 4, dans laquelle la saponine et le phosphatide de type monoacyle sont contenus chacun en une proportion de 0,1 à 5 % en poids, relativement au poids total de la composition cosmétique.

6. Composition cosmétique selon l'une quelconque des revendications 1 à 5, la composition cosmétique limpide étant choisie parmi les lotions pour le traitement de la peau, les préparations rafraîchissantes pour le visage, les lotions adoucissantes, les lotions pour le traitement de l'acné, les lotions après-rasage, les lotions de traitement contenant des pigments ou une poudre, les lotions démaquillantes et

les toniques capillaires.